# EUROPEAN PATENT APPLICATION

(11) **EP 4 530 284 A1**
(43) Date of publication of application: **02.04.2025**
(21) Application number: 24201349.8
(22) Date of filing: 19.09.2024
(51) Int. Cl.: C07D 233/58, C07D 233/60, C07C 53/10

(54) **METHOD FOR PRODUCING ONIUM SALT AND METHOD FOR PURIFYING ONIUM SALT**

(30) Priority: 28.09.2023 JP 2023168612
(71) Applicant: Koei Chemical Company, Limited, Sodegaura-shi, Chiba 299-0266 (JP)
(72) Inventor: TAKECHI, Yoshiaki, Chiba, 2990266 (JP); TAMAKOSHI, Satomi, Chiba, 2990266 (JP)
(74) Representative: Müller-Boré & Partner Patentanwälte PartG mbB

(57) **Abstract**

The present invention provides a method for producing an onium salt that is free of carboxylic acid or an onium salt with a decreased carboxylic acid content to such an extent that sufficient solubility of cellulose in the onium salt is achieved. The method for obtaining an onium salt from a mixture containing the onium salt and a carboxylic acid includes the following steps (1), (2) and (3): step (1) of obtaining a mixture containing an onium salt and a carboxylic acid, step (2) of adding an amine to the mixture containing the onium salt and the carboxylic acid to form an amine-carboxylic acid salt formed of the amine and the carboxylic acid, and step (3) of separating the onium salt from the amine-carboxylic acid salt produced in step (1) to obtain the onium salt.

## Description

### Technical Field

The present invention relates to a method for producing an onium salt and a method for purifying an onium salt.

### Background Art

Polysaccharides are substances composed of many monosaccharide molecules polymerized via glycosidic linkages. Among polysaccharides, cellulose, which is a polysaccharide composed of β-glucose molecules, is the most abundant biomass resource on Earth and is widely studied for its properties, such as dissolution and moldability, as an environmentally friendly material. However, cellulose is difficult to dissolve in common solvents because of the strong hydrogen bonds between or within the molecular chains.

Onium salts have been proposed as a solvent for dissolving cellulose. PTL 1 discloses a method for dissolving cellulose by using a molten ionic liquid composed of a 1-alkyl-3-methyl-imidazolium cation and an acetate anion.

PTL 2 discloses a method for producing a quaternary ammonium organic acid salt by producing a quaternary ammonium carbonate and mixing the produced quaternary ammonium carbonate with an organic acid to perform anion exchange on the organic acid. PTL 2 discloses a method for producing a quaternary ammonium organic acid salt by producing a quaternary ammonium carbonate from a nitrogen-containing heterocyclic aromatic compound, such as N-methylimidazole or N-methylbenzimidazole (starting material), mixing it with acetic acid (organic acid), and performing anion exchange.

PTL 3 discloses a method for producing a quaternary ammonium carboxylate by reacting a quaternary ammonium alkyl carbonate with carboxylic acid. In the Examples, PTL 3 discloses that N,N-diethyl-N-(2-methoxyethyl)-N-methylammonium=methylcarbonate was reacted with methoxyacetic acid to obtain N,N-diethyl-N-(2-methoxyethyl)-N-methylammonium=methoxyacetate.

NPL 1 discloses a one-pot synthesis process for 1,3-dialkylimidazolium acetate (when R₁ and R₂ are the same) and a mixture of 1-alkyl-3-methylimidazolium acetate, 1,3-dialkylimidazolium acetate, and 1,3-dimethylimidazolium acetate (when R₁ is a methyl group, and R₁ and R₂ are different), which is represented by the following scheme. NPL 1 also discloses that colored impurities can be removed by running ionic liquids, such as imidazolium acetate, through a decolorizing charcoal column.

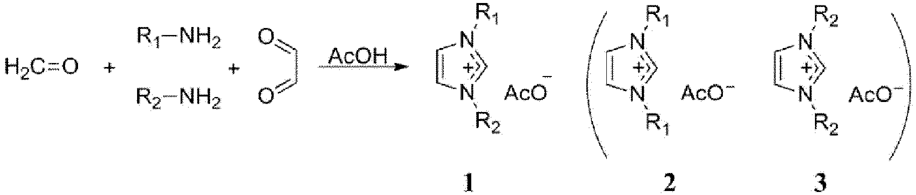

NPL 2 discloses a method for separating acetic acid from a synthetic mixture containing 1-ethyl-3-methylimidazolium acetate, which is an ionic liquid. The separation method described includes a method in which acetic acid is evaporated as a volatile component from a mixture containing 1-ethyl-3-methylimidazolium acetate, a method in which acetic acid is extracted with a solvent that is immiscible with 1-ethyl-3-methylimidazolium acetate, and a method in which methanol is added to a mixture containing 1-ethyl-3-methylimidazolium acetate to esterify acetic acid, and then methyl acetate is removed by distillation.

### Citation List

### Patent Literature

PTL 1: JP2005-506401A
PTL 2: JPS63-280045A
PTL 3: JP2015-83545A

### Non-patent Literature

NPL 1: Pure Appl. Chem., Vol. 84, No. 3, pp. 745-754, 2012
NPL 2: J. Chem. Eng. Data, 2013, 58, 197-202

### Summary of the Invention

### Technical Problem

In the methods for producing a quaternary ammonium carboxylate (an ionic liquid and also an onium salt) described in the prior art documents, the carboxylic acid used remains in the quaternary ammonium carboxylate (product). The remaining carboxylic acid forms an associate with the produced quaternary ammonium carboxylate. Using the quaternary ammonium carboxylate containing the associate in dissolving cellulose results in low solubility of cellulose in the quaternary ammonium carboxylate.

Given the current status of prior art, an object of the present invention is to provide a method for producing an onium salt that is free of carboxylic acid or an onium salt with a decreased carboxylic acid content to such an extent that sufficient solubility of cellulose in the onium salt is achieved. Another object of the present invention is to provide a method for purifying an onium salt, comprising removing carboxylic acid contained in the onium salt or decreasing the content of carboxylic acid to such an extent that sufficient solubility of cellulose in the onium salt is achieved.

### Solution to Problem

The present inventors conducted extensive research into methods for producing an onium salt that is free of carboxylic acid or an onium salt with a decreased carboxylic acid content to such an extent that sufficient solubility of cellulose in the onium salt is achieved, and also into methods for purifying an onium salt, and they completed the present invention.

Specifically, the present invention provides the following [1] to [10].
[1] A method for producing an onium salt represented by the following Formula (1): 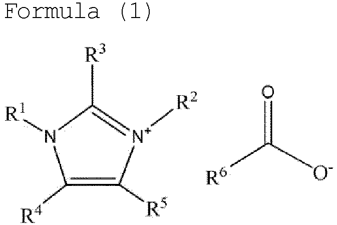 wherein
   R³, R⁴, and R⁵ may be identical or different, and each represents a hydrogen atom or a C₁-C₄ hydrocarbon group,
   R¹ and R² may be identical or different, and each represents a hydrocarbon group optionally containing at least one heteroatom,
   R¹ and R² may contain a branched and/or cyclic structure and/or a double bond,
   some or all of R¹, R², R³, R⁴, and R⁵, taken together with the nitrogen and/or carbon atoms to which R¹, R², R³, R⁴, and R⁵ are bonded, may form a cyclic structure, and
   R⁶ represents a hydrocarbon group optionally containing at least one heteroatom and may contain a double bond and/or a cyclic structure and/or a branched structure,
   the method comprising the following steps (1), (2), and (3) :
      step (1) of obtaining a mixture containing the onium salt represented by Formula (1) and a carboxylic acid represented by the following Formula (3), the mixture being obtained according to a reaction scheme represented by the following Formula (2): 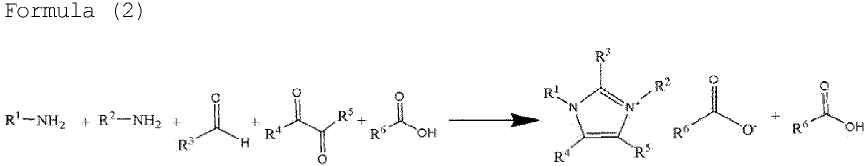 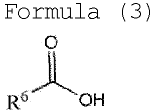 wherein R⁶ represents a hydrocarbon group optionally containing at least one heteroatom and may contain a double bond and/or a cyclic structure and/or a branched structure,
      step (2) of adding an amine to the mixture containing the onium salt represented by Formula (1) and the carboxylic acid represented by Formula (3) obtained in step (1) to generate an amine-carboxylic acid salt formed of the amine and the carboxylic acid represented by Formula (3), and
      step (3) of separating the onium salt represented by Formula (1) from the amine-carboxylic acid salt generated in step (2) to obtain the onium salt represented by Formula (1).
[2] The method for producing an onium salt according to [1], wherein the amine added in step (2) is an amine represented by the following Formula (4): 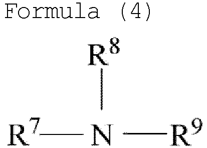 wherein
   R⁷, R⁸, and R⁹ may be identical or different, and each represents a hydrogen atom or a C₁-C₁₈ hydrocarbon group,
   the C₁-C₁₈ hydrocarbon group may contain a double bond and may contain a branched structure and/or a cyclic structure, and
   the total number of carbon atoms contained in each of R⁷, R⁸, and R⁹ is 8 or greater.
[3] The method for producing an onium salt according to [1], wherein the onium salt represented by Formula (1) is of at least two types.
[4] The method for producing an onium salt according to [1], wherein the mixture containing the onium salt represented by Formula (1) and the carboxylic acid represented by Formula (3) further contains water and/or a hydrophilic solvent.
[5] The method for producing an onium salt according to [1], wherein the amine added in step (2) is an amine solution prepared by diluting the amine with a solvent.
[6] A method for purifying an onium salt represented by the following Formula (1), comprising removing a carboxylic acid represented by the following Formula (3) from a mixture containing the onium salt represented by Formula (1) and the carboxylic acid represented by Formula (3), 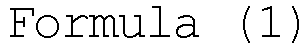 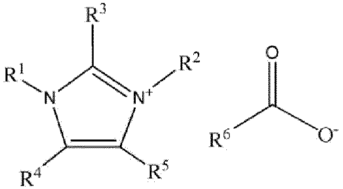 wherein
   R³, R⁴, and R⁵ may be identical or different, and each represents a hydrogen atom or a C₁-C₄ hydrocarbon group,
   R¹ and R² may be identical or different, and each represents a hydrocarbon group optionally containing at least one heteroatom,
   R¹ and R² may contain a branched and/or cyclic structure and/or a double bond,
   some or all of R¹, R², R³, R⁴, and R⁵, taken together with the nitrogen and/or carbon atoms to which R¹, R², R³, R⁴, and R⁵ are bonded, may form a cyclic structure, and
   R⁶ represents a hydrocarbon group optionally containing at least one heteroatom and may contain a double bond and/or a cyclic structure and/or a branched structure,
   the method comprising the following steps (1), (2) and (3) :
      step (1) of obtaining a mixture containing the onium salt represented by Formula (1) and the carboxylic acid represented by Formula (3), the mixture being obtained according to a reaction scheme represented by the following Formula (2): 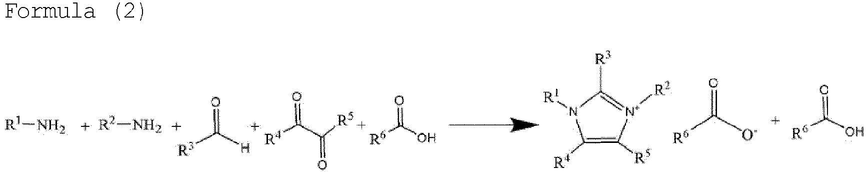 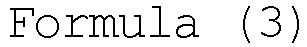 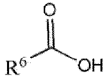 wherein R⁶ represents a hydrocarbon group optionally containing at least one heteroatom and may contain a double bond and/or a cyclic structure and/or a branched structure,
      step (2) of adding an amine to the mixture containing the onium salt represented by Formula (1) and the carboxylic acid represented by Formula (3) obtained in step (1) to generate an amine-carboxylic acid salt formed of the amine and the carboxylic acid represented by Formula (3), and
      step (3) of separating the onium salt represented by Formula (1) from the amine-carboxylic acid salt generated in step (2) to obtain the onium salt represented by Formula (1).
[7] The method for purifying an onium salt according to [6], wherein the amine added in step (2) is an amine represented by the following Formula (4): 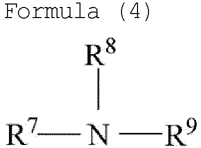 wherein
   R⁷, R⁸, and R⁹ may be identical or different, and each represents a hydrogen atom or a C₁-C₁₈ hydrocarbon group,
   the Cₗ-C₁₈ hydrocarbon group may contain a double bond and may contain a branched structure and/or a cyclic structure, and
   the total number of carbon atoms contained in each of R⁷, R⁸, and R⁹ is 8 or greater.
[8] The method for purifying an onium salt according to [6], wherein the onium salt represented by Formula (1) is of at least two types.
[9] The method for purifying an onium salt according to [6], wherein the mixture containing the onium salt represented by Formula (1) and the carboxylic acid represented by Formula (3) further contains water and/or a hydrophilic solvent.
[10] The method for purifying an onium salt according to [6], wherein the amine added in step (2) is an amine solution prepared by diluting the amine with a solvent.

### Advantageous Effects of Invention

The present invention provides a method for producing an onium salt that is free of carboxylic acid or an onium salt with a decreased carboxylic acid content to such an extent that sufficient solubility of cellulose in the onium salt is achieved. The present invention also provides a method for purifying an onium salt, comprising removing carboxylic acid contained in the onium salt or decreasing the content of carboxylic acid to such an extent that sufficient solubility of cellulose in the onium salt is achieved.

### Description of Embodiments

The present invention is specifically described below. One embodiment of the invention relates to a method for producing an onium salt represented by the following Formula (1): 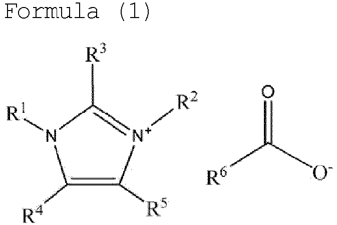 wherein
R³, R⁴, and R⁵ may be identical or different, and each represents a hydrogen atom or a C₁-C₄ hydrocarbon group,
R¹ and R² may be identical or different, and each represents a hydrocarbon group optionally containing at least one heteroatom,
R¹ and R² may contain a branched and/or cyclic structure and/or a double bond,
some or all of R¹, R², R³, R⁴, and R⁵, taken together with the nitrogen and/or carbon atoms to which R¹, R², R³, R⁴, and R⁵ are bonded, may form a cyclic structure, and
R⁶ represents a hydrocarbon group optionally containing at least one heteroatom and may contain a double bond and/or a cyclic structure and/or a branched structure,
the method comprising the following steps (1), (2), and (3)
   step (1) of obtaining a mixture containing the onium salt represented by Formula (1) and a carboxylic acid represented by the following Formula (3), the mixture being obtained according to a reaction scheme represented by the following Formula (2): 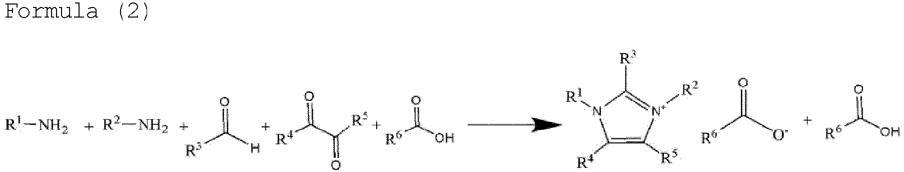 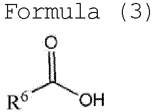 wherein R⁶ represents a hydrocarbon group optionally containing at least one heteroatom and may contain a double bond and/or a cyclic structure and/or a branched structure,
   step (2) of adding an amine to the mixture containing the onium salt represented by Formula (1) and the carboxylic acid represented by Formula (3) obtained in step (1) to generate an amine-carboxylic acid salt formed of the amine and the carboxylic acid represented by Formula (3), and
   step (3) of separating the onium salt represented by Formula (1) from the amine-carboxylic acid salt generated in step (2) to obtain the onium salt represented by Formula (1).

The onium salt represented by the following Formula (1) is an imidazolium carboxylate containing an imidazolium cation and a carboxylate anion. 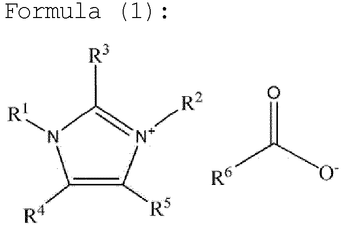 wherein
R³, R⁴, and R⁵ may be identical or different, and each represents a hydrogen atom or a C₁-C₄ hydrocarbon group,
R¹ and R² may be identical or different, and each represents a hydrocarbon group optionally containing at least one heteroatom,
R¹ and R² may contain a branched and/or cyclic structure and/or a double bond,
some or all of R¹, R², R³, R⁴, and R⁵, taken together with the nitrogen and/or carbon atoms to which R¹, R², R³, R⁴, and R⁵ are bonded, may form a cyclic structure, and
R⁶ represents a hydrocarbon group optionally containing at least one heteroatom and may contain a double bond and/or a cyclic structure and/or a branched structure.

The imidazolium cation represented by the following Formula (1-1) in Formula (1) is explained. 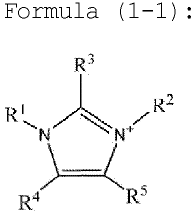 wherein
R³, R⁴, and R⁵ may be identical or different, and each represents a hydrogen atom or a C₁-C₄ hydrocarbon group,
R¹ and R² may be identical or different, and each represents a hydrocarbon group optionally containing at least one heteroatom,
R¹ and R² may contain a branched and/or cyclic structure and/or a double bond, and
some or all of R¹, R², R³, R⁴, and R⁵, taken together with the nitrogen and/or carbon atoms to which R¹, R², R³, R⁴, and R⁵ are bonded, may form a cyclic structure.

In Formula (1-1), R¹ and R² each represent a hydrocarbon group optionally containing at least one heteroatom. The "hydrocarbon group optionally containing at least one heteroatom" may be a linear chain and may contain a branched and/or cyclic structure and/or a double bond. The hydrocarbon groups represented by R¹ and R² may contain no heteroatom.

In Formula (1-1), when R¹ and R² are each at least one hydrocarbon group containing no heteroatom, the number of carbon atoms is preferably 1 to 12, more preferably 1 to 8, and even more preferably 1 to 3. When R¹ and R² are each a hydrocarbon group containing a heteroatom, the number of carbon atoms is preferably 2 to 12, more preferably 2 to 8, and even more preferably 2 to 4.

In Formula (1-1), when R¹ and R² each contain at least one heteroatom, the number of heteroatoms is preferably 1 to 5, and more preferably 1 or 2. The number of carbon atoms in the hydrocarbon group is two or more, preferably 2 to 12, more preferably 2 to 8, and even more preferably 2 to 4.

In Formula (1-1), when R¹ and R² are each a hydrocarbon group containing at least one heteroatom, examples of heteroatoms include a nitrogen atom, an oxygen atom, and a sulfur atom. The number of heteroatoms is preferably 1 to 5, and more preferably 1 or 2.

In Formula (1-1), the hydrocarbon group containing at least one heteroatom in R¹ and R² refers to a hydrocarbon group formed by replacing (i) the bond between two adjacent carbon atoms and/or (ii) the hydrogen atom of one -CH₂- group (methylene group) and the hydrogen atom of another -CH₂- group (methylene group) in a hydrocarbon group containing no heteroatom with a structure such as -O-, -N<, -NH-, -S-, -C(=O)-O-, -C(=O)-, =N-, - C(=O)-N<, -C(=O)-NH-, -O-C(=O)-O-, -O-C(=O)-N<, -O-C(=O)-NH-, - C(=O)-N[-C(=O)-]-, -C(=O)-NH-C(=O)-, >N-C(=O)-N<, >N-C(=O)-NH-, - HN-C(=O)-NH-, -S(=O)-, -S(=O)₂-, -S(=O)₂-O-, -O-S(=O)₂-O-, >N-C(=S)-N<, >N-C(=S)-NH-, -HN-C(=S)-NH-, -C(=N-)-, or -C(=NH)-. The number and combination of replacements by these structures are not particularly limited; however, the number of heteroatoms in R¹ and R² is preferably 1 to 5, and more preferably 1 or 2.

Preferably, the hydrocarbon group containing a heteroatom is a hydrocarbon group formed by replacing (i) the bond between two adjacent carbon atoms and/or (ii) the hydrogen atom of one -CH₂- group (methylene group) and the hydrogen atom of another -CH₂- (methylene group) with -O- or -S-, and more preferably with -O-.

In the present invention, the structure in which (i) the bond between two adjacent carbon atoms and/or (ii) the hydrogen atom of one -CH₂- (methylene group) and the hydrogen atom of another -CH₂- (methylene group) are replaced with -O- or -S-may be not only an ether structure or a thioether structure, but also be an epoxy structure or a thioepoxy structure, such as an epoxy group, a thioepoxy group, a glycidyl ether group, or an epoxycyclohexyl group. In this case, R¹ and R² may be, for example, an epoxy group or a thioepoxy group.

When R¹ and R² contain two or more structures of -O- and/or -S-, the number of carbon atoms between the structures of -O- and/or -S- is preferably two or more.

Examples of the hydrocarbon group optionally containing at least one heteroatom represented by R¹ and R² in Formula (1-1) include a linear or branched alkyl group optionally containing at least one heteroatom, a linear or branched alkenyl group optionally containing at least one heteroatom, a linear or branched alkynyl group optionally containing at least one heteroatom, a cycloalkyl group optionally branched and optionally containing at least one heteroatom, a cycloalkenyl group optionally branched and optionally containing at least one heteroatom, an aryl group optionally branched and optionally containing at least one heteroatom, and an aralkyl group optionally branched and optionally containing at least one heteroatom. Preferred are a linear or branched alkyl group optionally containing at least one heteroatom, a linear or branched alkenyl group optionally containing at least one heteroatom, and a cycloalkyl group optionally branched and optionally containing at least one heteroatom.

The "linear or branched alkyl group optionally containing at least one heteroatom" is preferably a linear or branched C₁-C₁₂ alkyl group optionally containing 1 to 5 heteroatoms. In another embodiment, the linear or branched alkyl group optionally containing at least one heteroatom is preferably a linear or branched C₁-C₁₂ alkyl group optionally containing 1 or 2 heteroatoms, more preferably a linear or branched C₁-C₈ alkyl group optionally containing 1 or 2 heteroatoms, and even more preferably a linear or branched C₁-C₄ alkyl group optionally containing 1 or 2 heteroatoms. In yet another embodiment, the linear or branched alkyl group optionally containing at least one heteroatom is preferably a linear or branched C₁-C₁₂ alkyl group, more preferably a linear or branched C₁-C₈ alkyl group, and even more preferably a linear or branched C₁-C₃ alkyl group.

The "linear or branched alkenyl group optionally containing at least one heteroatom" is preferably a linear or branched C₂-C₁₂ alkenyl group optionally containing 1 to 5 heteroatoms. In another embodiment, the linear or branched alkenyl group optionally containing at least one heteroatom is preferably a linear or branched C₂-C₁₂ alkenyl group optionally containing 1 or 2 heteroatoms, more preferably a linear or branched C₂-C₈ alkenyl group optionally containing 1 or 2 heteroatoms, and even more preferably a linear or branched C₂-C₄ alkenyl group optionally containing 1 or 2 heteroatoms. In yet another embodiment, the linear or branched alkenyl group optionally containing at least one heteroatom is preferably a linear or branched C₂-C₁₂ alkenyl group, more preferably a linear or branched C₂-C₈ alkenyl group, and even more preferably a linear or branched C₂-C₃ alkenyl group.

The "cycloalkyl group optionally branched and optionally containing at least one heteroatom" is preferably a C₃-C₁₂ cycloalkyl group optionally branched and optionally containing 1 to 5 heteroatoms. In another embodiment, the cycloalkyl group optionally branched and optionally containing at least one heteroatom is preferably a C₃-C₁₂ cycloalkyl group optionally branched and optionally containing 1 or 2 heteroatoms, more preferably a C₃-C₈ cycloalkyl group optionally branched and optionally containing 1 or 2 heteroatoms, and even more preferably a C₃-C₄ cycloalkyl group optionally branched and optionally containing 1 or 2 heteroatoms. In yet another embodiment, the cycloalkyl group optionally branched and optionally containing a heteroatom is preferably an optionally branched C₃-C₁₂ cycloalkyl group, more preferably an optionally branched C₃-C₈ cycloalkyl group, and even more preferably a C₃ cycloalkyl group.

Specific examples of the hydrocarbon groups containing no heteroatom represented by R¹ and R² in Formula (1-1) include a methyl group, an ethyl group, a propyl group, an isopropyl group, a butyl group, a sec-butyl group, an isobutyl group, a tert-butyl group, a pentyl group, a neopentyl group, a hexyl group, an octyl group, a 2-ethylhexyl group, a decyl group, a dodecyl group, a vinyl group, an ethynyl group, a propenyl group, an isopropenyl group, an allyl group, a propargyl group, a butenyl group, a crotyl group, a butadienyl group, a cyclopropyl group, a cyclobutyl group, a cyclopentyl group, a cyclohexyl group, a norbornyl group, an adamantyl group, a phenyl group, a benzyl group, a tolyl group, a styryl group, and a 2,4,6-trimethylphenyl group. The hydrocarbon groups containing no heteroatom are preferably a methyl group, an ethyl group, a propyl group, an isopropyl group, a butyl group, a sec-butyl group, an isobutyl group, a tert-butyl group, a pentyl group, a neopentyl group, and a hexyl group, and more preferably a methyl group, an ethyl group, a propyl group, an isopropyl group, and a tert-butyl group.

The "hydrocarbon group containing a heteroatom" represented by R¹ and R² In Formula (1-1) is preferably a 3-indolyl group, a p-methoxyphenyl group, a 7-oxabicyclo[2,2,1]hept-5-en-2-yl group, a 1,2-epoxyethyl group, a 1-methyl-2-methoxyvinyl group, a 2-furyl group, a 2-pyridyl group, a 4-imidazolyl group, or a benzyloxymethyl group, and more preferably a 1-methyl-2-methoxyvinyl group or a 2-furyl group, although the present invention is not limited by these groups.

In Formula (1-1), R³, R⁴, and R⁵ are preferably a hydrogen atom or a methyl group, and more preferably a hydrogen atom.

In Formula (1-1), some or all of R¹, R², R³, R⁴, and R⁵, taken together with the nitrogen and/or carbon atoms to which R¹, R², R³, R⁴, and R⁵ are bonded, may form a cyclic structure. A specific example of the cyclic structure is, but is not limited to, a structure in which R¹ and R³, R³ and R², R² and R⁵, R⁵ and R⁴, or R¹ and R⁴ are bonded together and crosslinked with a C₂-C₂₄ hydrocarbon containing 2 to 10 heteroatoms to thereby form a ring structure.

Specific examples of the imidazolium having a hydrocarbon group containing no heteroatom in Formula (1-1) include 1,3-dimethylimidazolium, 1,3-diethylimidazolium, 1-ethyl-3-methylimidazolium, 1-methyl-3-propylimidazolium, 1-butyl-3-methylimidazolium, 1-methyl-3-pentylimidazolium, 1-hexyl-3-methylimidazolium, 1-heptyl-3-methylimidazolium, 1-methyl-3-octylimidazolium, 1-methyl-3-nonylimidazolium, 1-decyl-3-methylimidazolium, 1-allyl-3-methylimidazolium, and 1-allyl-3-ethylimidazolium. The imidazolium having a hydrocarbon group containing no heteroatom is preferably 1,3-dimethylimidazolium, 1-ethyl-3-methylimidazolium, 1,3-diethylimidazolium, and 1-butyl-3-methylimidazolium, although the present invention is not limited by these compounds.

Specific examples of the imidazolium having a hydrocarbon group containing a heteroatom in Formula (1-1) include 1,3-bis(2-methoxyethyl)imidazolium, 1,3-bis(2-ethoxyethyl)imidazolium, 1,3-bis(2-propoxyethyl)imidazolium, 1,3-bis(2-isopropoxyethyl)imidazolium, 1,3-bis(2-phenoxyethyl)imidazolium, 1,3-bis(2-benzyloxyethyl)imidazolium, 1,3-bis[2-(p-methoxybenzyloxy)ethyl]imidazolium, 1,3-bis[2-(2-methoxyethoxymethoxy)ethyl]imidazolium, 1,3-bis[2-(2-benzyloxyethoxy)ethyl]imidazolium, 1,3-bis(3-methoxypropyl)imidazolium, 1,3-bis(3-ethoxypropyl)imidazolium, 1,3-bis(3-propoxypropyl)imidazolium, 1,3-bis(3-isopropoxypropyl)imidazolium, 1,3-bis(3-allyloxypropyl)imidazolium, 1,3-bis(3-butoxypropyl)imidazolium, 1,3-bis[3-(1-methylpropoxy)propyl]imidazolium, 1,3-bis[3-(2-methylpropoxy)propyl]imidazolium, 1,3-bis[3-(1,1-dimethylethoxy)propyl]imidazolium, 1,3-bis[(oxolan-2-yl)methyl]imidazolium, 1,3-bis(3-pentyloxypropyl)imidazolium, 1,3-bis(3-neopentyloxypropyl)imidazolium, 1,3-bis(3-hexyloxypropyl)imidazolium, 1,3-bis(3-cyclohexyloxypropyl)imidazolium, 1,3-bis(3-heptyloxypropyl)imidazolium, 1,3-bis(3-octyloxypropyl)imidazolium, 1,3-bis(3-nonyloxypropyl)imidazolium, 1,3-bis{3-[(2-ethylhexan-1-yl)oxy]propyl}imidazolium, 1,3-bis[3-(2,4,6-trimethylphenoxy)propyl]imidazolium, 1,3-bis[3-(2-methoxyethoxy)propyl]imidazolium, 1,3-bis[2-(dimethylamino)ethyl]imidazolium, 1,3-bis[3-(dimethylamino)propyl]imidazolium, 1,3-bis(2-methylthioethyl)imidazolium, 1,3-bis{2-[(furan-2-yl)methylthio]ethyl]imidazolium, 1,3-bis[2-(furan-2-yl)methyl]imidazolium, 1,3-bis[1-(methoxymethyl)propyl]imidazolium, 1,3-bis(4-methoxybutyl)imidazolium, 1,3-bis(4-ethoxybutyl)imidazolium, 1,3-bis(4-propoxybutyl)imidazolium, 1,3-bis(4-isopropoxybutyl)imidazolium, 1,3-bis(4-allyloxybutyl)imidazolium, 1,3-bis(4-butoxybutyl)imidazolium, 1,3-bis[4-(1-methylpropoxy)butyl]imidazolium, 1,3-bis[4-(2-methylpropoxy)butyl]imidazolium, 1,3-bis[4-(1,1-dimethylethoxy)butyl]imidazolium, 1,3-bis(4-pentyloxybutyl)imidazolium, 1,3-bis(4-neopentyloxybutyl)imidazolium, 1,3-bis(4-hexyloxybutyl)imidazolium, 1,3-bis(4-cyclohexyloxybutyl)imidazolium, 1,3-bis(4-heptyloxybutyl)imidazolium, 1,3-bis(4-octyloxybutyl)imidazolium, 1,3-bis{4-[(2-ethylhexan-1-yl)oxy]butyl}imidazolium, 1,3-bis(5-methoxypentyl)imidazolium, 1,3-bis(5-ethoxypentyl)imidazolium, 1,3-bis(5-propoxypentyl)imidazolium, 1,3-bis(5-isopropoxypentyl)imidazolium, 1,3-bis(5-allyloxypentyl)imidazolium, 1,3-bis(5-butoxypentyl)imidazolium, 1,3-bis[5-(1-methylpropoxy)pentyl]imidazolium, 1,3-bis[5-(2-methylpropoxy)pentyl]imidazolium, 1,3-bis[5-(1,1-dimethylethoxy)pentyl]imidazolium, 1,3-bis(5-pentyloxypentyl)imidazolium, 1,3-bis(5-neopentyloxypentyl)imidazolium, 1,3-bis(5-hexyloxypentyl)imidazolium, 1,3-bis(5-cyclohexyloxypentyl)imidazolium, 1,3-bis(5-heptyloxypentyl)imidazolium, 1,3-bis(6-methoxyhexyl)imidazolium, 1,3-bis(6-ethoxyhexyl)imidazolium, 1,3-bis(6-propoxyhexyl)imidazolium, 1,3-bis(6-isopropoxyhexyl)imidazolium, 1,3-bis(6-allyloxyhexyl)imidazolium, 1,3-bis(6-butoxyhexyl)imidazolium, 1,3-bis[6-(1-methylpropoxy)hexyl]imidazolium, 1,3-bis[6-(2-methylpropoxy)hexyl]imidazolium, 1,3-bis[6-(1,1-dimethylethoxy)hexyl]imidazolium, 1,3-bis(6-pentyloxyhexyl)imidazolium, 1,3-bis(6-neopentyloxyhexyl)imidazolium, 1,3-bis(6-hexyloxyhexyl)imidazolium, 1,3-bis(6-cyclohexyloxyhexyl)imidazolium, 1,3-bis[(2-methoxycyclohexan-1-yl)methyl]imidazolium, 1,3-bis[(2-ethoxycyclohexan-1-yl)methyl]imidazolium, 1,3-bis[(2-propoxycyclohexan-1-yl)methyl]imidazolium, 1,3-bis[(2-isopropoxycyclohexan-1-yl)methyl]imidazolium, 1,3-bis[(2-butoxycyclohexan-1-yl)methyl]imidazolium, 1,3-bis{[2-(1-methylpropoxy)cyclohexan-1-yl]methyl}imidazolium, 1,3-bis{[2-(2-methylpropoxy)cyclohexan-1-yl]methyl}imidazolium, 1,3-bis{[2-(1,1-dimethylethoxy)cyclohexan-1-yl]methyl}imidazolium, 1,3-bis[(2-pentyloxycyclohexan-1-yl)methyl]imidazolium, 1,3-bis[(2-neopentyloxycyclohexan-1-yl)methyl]imidazolium, 1,3-bis(3-methylthiopropyl)imidazolium, 1,3-bis[2-(methylsulfonyl)ethyl]imidazolium, 1,3-bis[(thiophen-2-yl)methyl]imidazolium, 1,3-bis(p-methoxyphenyl)imidazolium, 1,3-bis(p-ethoxyphenyl)imidazolium, 1,3-bis(3,4-methylenedioxyphenyl)imidazolium, 1,3-bis(3,4-methylenedioxybenzyl)imidazolium, 1,3-bis{(7-oxabicyclo[2,2,1]heptan-2-yl)methyl}imidazolium, 1-methoxypropyl-3-methylthiopropyl imidazolium, 1,3-bis(3-methoxypropyl)-2-methylimidazolium, and 1,3-bis(2-methoxyethyl)-2-methylimidazolium. The imidazolium having a hydrocarbon group containing a heteroatom is preferably 1,3-bis(3-methoxypropyl)imidazolium, 1,3-bis(3-ethoxypropyl)imidazolium, 1,3-bis(3-propoxypropyl)imidazolium, 1,3-bis(3-isopropoxypropyl)imidazolium, 1,3-bis(3-allyloxypropyl)imidazolium, 1,3-bis(3-butoxypropyl)imidazolium, 1,3-bis[3-(1-methylpropoxy)propyl]imidazolium, 1,3-bis[3-(2-methylpropoxy)propyl]imidazolium, 1,3-bis[3-(1,1-dimethylethoxy)propyl]imidazolium, 1,3-bis[(oxolan-2-yl)methyl]imidazolium, 1,3-bis[3-(2-methoxyethoxy)propyl]imidazolium, 1,3-bis(3-methylthiopropyl)imidazolium, 1-methoxypropyl-3-methylthiopropyl imidazolium, 1,3-bis(3-methoxypropyl)-2-methylimidazolium, or 1,3-bis(2-methoxyethyl)-2-methylimidazolium, more preferably 1,3-bis(3-methoxypropyl)imidazolium, 1,3-bis(3-ethoxypropyl)imidazolium, 1-methoxypropyl-3-methylthiopropyl imidazolium, 1,3-bis[3-(2-methoxyethoxy)propyl]imidazolium, 1,3-bis(2-methoxyethyl)-2-methylimidazolium, or 1,3-bis(3-methoxypropyl)-2-methylimidazolium, although the present invention is not limited by these compounds.

The carboxylate anion represented by the following Formula (1-2) in Formula (1) is explained. 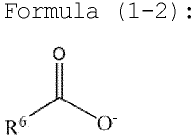 wherein R⁶ represents a hydrocarbon group optionally containing at least one heteroatom and may contain a double bond and/or a cyclic structure and/or a branched structure.

In Formula (1-2), R⁶ represents a hydrocarbon group optionally containing at least one heteroatom. The "hydrocarbon group optionally containing at least one heteroatom" may be a linear chain, or may contain a branched and/or cyclic structure and/or a double bond. Additionally, the hydrocarbon group represented by R⁶ may contain no heteroatom.

In Formula (1-2), when R⁶ is a hydrocarbon group containing no heteroatom, the number of carbon atoms is preferably 1 to 12, more preferably 1 to 8, and even more preferably 1 to 3. When R⁶ is a hydrocarbon group containing a heteroatom, the number of carbon atoms is preferably 2 to 12, more preferably 2 to 8, and even more preferably 2 to 4.

In Formula (1-2), when R⁶ contains at least one heteroatom, the number of heteroatoms is preferably 1 to 5, and more preferably 1 or 2. The number of carbon atoms of the hydrocarbon group is 2 or more, preferably 2 to 12, more preferably 2 to 8, and even more preferably 2 to 4.

When R⁶ is a hydrocarbon group containing at least one heteroatom in Formula (1-2), examples of heteroatoms include a nitrogen atom, an oxygen atom, and a sulfur atom. The number of heteroatoms is preferably 1 to 5, and more preferably 1 or 2.

In Formula (1-2), the "hydrocarbon group containing at least one heteroatom" for R⁶ refers to a hydrocarbon group formed by replacing (i) the bond between two adjacent carbon atoms and/or (ii) the hydrogen atom of one -CH₂- group (methylene group) and the hydrogen atom of another -CH₂- group (methylene group) in a hydrocarbon group containing no heteroatom with a structure such as -O-, -N<, -NH-, -S-, -C(=O)-O-, -C(=O)-, =N-, -C(=O)-N<, -C(=O)-NH-, -O-C(=O)-O-, -O-C(=O)-N<, -O-C(=O)-NH-, -C(=O)-N[-C(=O)-]-, -C(=O)-NH-C(=O)-, >N-C(=O)-N<, >N-C(=O)-NH-, -HN-C(=O)-NH-, -S(=O)-, -S(=O)₂-, -S(=O)₂-O-, -O-S(=O)₂-O-, >N-C(=S)-N<, >N-C(=S)-NH-, -HN-C(=S)-NH-, -C(=N-)-, or -C(=NH)-. The number and combination of replacements by these structures are not particularly limited; however, the number of heteroatoms in R⁶ is preferably 1 to 5, and more preferably 1 or 2.

Preferably, the hydrocarbon group containing at least one heteroatom is a hydrocarbon group formed by replacing (i) the bond between two adjacent carbon atoms and/or (ii) the hydrogen atom of one -CH₂- group (methylene group) and the hydrogen atom of another -CH₂- group (methylene group) with -O- or -S-, and more preferably with -O-.

In R⁶ in Formula (1-2), the structure in which (i) the bond between two adjacent carbon atoms and/or (ii) the hydrogen atom of one -CH₂- (methylene group) and the hydrogen atom of another -CH₂- (methylene group) are replaced with -O- or -S- may be not only an ether structure or a thioether structure, but also an epoxy structure or a thioepoxy structure, such as an epoxy group, a thioepoxy group, a glycidyl ether group, or an epoxycyclohexyl group. In this case, R¹ may be, for example, an epoxy group or a thioepoxy group.

In Formula (1-2), when R⁶ contains two or more structures of -O- and/or -S-, the number of carbon atoms between the structures of -O- and/or -S- is preferably two or more.

Examples of the hydrocarbon group optionally containing at least one heteroatom in R⁶ in Formula (1-2) include a linear or branched alkyl group optionally containing at least one heteroatom, a linear or branched alkenyl group optionally containing at least one heteroatom, a linear or branched alkynyl group optionally containing at least one heteroatom, a cycloalkyl group optionally branched and optionally containing at least one heteroatom, a cycloalkenyl group optionally branched and optionally containing at least one heteroatom, an aryl group optionally branched and optionally containing at least one heteroatom, and an aralkyl group optionally branched and optionally containing at least one heteroatom. Preferred are a linear or branched alkyl group optionally containing at least one heteroatom, a linear or branched alkenyl group optionally containing at least one heteroatom, and a cycloalkyl group optionally branched and optionally containing at least one heteroatom.

The "linear or branched alkyl group optionally containing at least one heteroatom" is preferably a linear or branched C₁-C₁₂ alkyl group optionally containing 1 to 5 heteroatoms. In another embodiment, the linear or branched alkyl group optionally containing at least one heteroatom is preferably a linear or branched C₁-C₁₂ alkyl group optionally containing 1 or 2 heteroatoms, more preferably a linear or branched C₁-C₈ alkyl group optionally containing 1 or 2 heteroatoms, and even more preferably a linear or branched C₁-C₄ alkyl group optionally containing 1 or 2 heteroatoms. In yet another embodiment, the linear or branched alkyl group optionally containing at least one heteroatom is preferably a linear or branched C₁-C₁₂ alkyl group, more preferably a linear or branched C₁-C₈ alkyl group, and even more preferably a linear or branched C₁-C₃ alkyl group.

The "linear or branched alkenyl group optionally containing at least one heteroatom" is preferably a linear or branched C₂-C₁₂ alkenyl group optionally containing 1 to 5 heteroatoms. In another embodiment, the linear or branched alkenyl group optionally containing at least one heteroatom is preferably a linear or branched C₂-C₁₂ alkenyl group optionally containing 1 or 2 heteroatoms, more preferably a linear or branched C₂-C₈ alkenyl group optionally containing 1 or 2 heteroatoms, and even more preferably a linear or branched C₂-C₄ alkenyl group optionally containing 1 or 2 heteroatoms. In yet another embodiment, the linear or branched alkenyl group optionally containing at least one heteroatom is preferably a linear or branched C₂-C₁₂ alkenyl group, more preferably a linear or branched C₂-C₈ alkenyl group, and even more preferably a linear or branched C₂-C₃ alkenyl group.

The "cycloalkyl group optionally branched and optionally containing at least one heteroatom" is preferably a C₃-C₁₂ cycloalkyl group optionally branched and optionally containing 1 to 5 heteroatoms. In another embodiment, the cycloalkyl group optionally branched and optionally containing a heteroatom is preferably a C₃-C₁₂ cycloalkyl group optionally branched and optionally containing 1 or 2 heteroatoms, more preferably a C₃-C₈ cycloalkyl group optionally branched and optionally containing 1 or 2 heteroatoms, and even more preferably a C₃-C₄ cycloalkyl group optionally branched and optionally containing 1 or 2 heteroatoms. In yet another embodiment, the cycloalkyl group optionally branched and optionally containing a heteroatom is preferably an optionally branched C₃-C₁₂ cycloalkyl group, more preferably an optionally branched C₃-C₈ cycloalkyl group, and even more preferably a C₃ cycloalkyl group.

Specific examples of the hydrocarbon group containing no heteroatom represented by R⁶ in Formula (1-2) include a methyl group, an ethyl group, a propyl group, an isopropyl group, a butyl group, a sec-butyl group, an isobutyl group, a tert-butyl group, a pentyl group, a neopentyl group, a hexyl group, an octyl group, a 2-ethylhexyl group, a decyl group, a dodecyl group, a vinyl group, an ethynyl group, a propenyl group, an isopropenyl group, an allyl group, a propargyl group, a butenyl group, a crotyl group, a butadienyl group, a cyclopropyl group, a cyclobutyl group, a cyclopentyl group, a cyclohexyl group, a norbornyl group, an adamantyl group, a phenyl group, a benzyl group, a tolyl group, a styryl group, and a 2,4,6-trimethylphenyl group. The hydrocarbon group containing no heteroatom is preferably a methyl group, an ethyl group, a propyl group, an isopropyl group, a butyl group, a sec-butyl group, an isobutyl group, a tert-butyl group, a pentyl group, a neopentyl group, and a hexyl group, and more preferably a methyl group, an ethyl group, a propyl group, an isopropyl group, and a tert-butyl group.

The "hydrocarbon group containing at least one heteroatom" represented by R⁶ in Formula (1-2) is preferably a methoxymethyl group, a methoxyethyl group, an ethoxymethyl group, a propoxy group, or a 2-methoxyethoxy group, and more preferably a methoxymethyl group, a methoxyethyl group, or a 2-methoxyethoxy group, although the present invention is not limited by these groups.

In Formula (1-2), R⁶ is preferably a methyl group, an ethyl group, a methoxymethyl group, or a methoxyethyl group.

Specific examples of the carboxylate anion represented by Formula (1-2) include an acetate ion, a propionate ion, an acrylate ion, propargylate ion, a butyrate ion, an isobutyrate ion, a methacrylate ion, a crotonate ion, a tetrolate ion, a cyclopropanecarboxylate ion, a valerate ion, an isovalerate ion, a pivalate ion, an angelicate ion, a tiglate ion, a cyclobutanecarboxylate ion, a caproate ion, a cyclopentanecarboxylate ion, an enanthate ion, a sorbate ion, a cyclohexanecarboxylate ion, a benzoate ion, a caprylate ion, a 2-ethylhexylcarboxylate ion, a toluate ion, a 2-norbornane carboxylate ion, a pelargonate ion, a cinnamate ion, a caprate ion, an adamantanecarboxylate ion, a geranate ion, an undecylate ion, a laurate ion, and a naphthalenecarboxylate ion. The carboxylate anion is preferably an acetate ion, a propionate ion, an acrylate ion, a propargylate ion, a butyrate ion, a isobutyrate ion, a methacrylate ion, a crotonate ion, a tetrolate ion, a valerate ion, an isovalerate ion, a pivalate ion, a methoxyacetate ion, an ethoxyacetate ion, a propoxyacetate ion, a (2-methoxyethoxy)acetate ion, a (2-ethoxyethoxy)acetate ion, a (2-propoxyethoxy)acetate ion, a 3-(2-methoxyethoxy)propanoate ion, a 3-(2-ethoxyethoxy)propanoate ion, a 3-(2-propoxyethoxy)propanoate ion, a 3-(3-methoxypropoxy)propanoate ion, a 3-(3-ethoxypropoxy)propanoate ion, a 3-(3-propoxypropoxy)propanoate ion, a 3-[2-(2-methoxyethoxy)ethoxy]propanoate ion, a 3-[2-(2-ethoxyethoxy)ethoxy]propanoate ion, a 4,7,10,13-tetraoxatetradecanoate ion, a 4,7,10,13-tetraoxapentadecanoate ion, a 4,7,10,13,16-pentaoxaheptadecanoate ion, a 4,7,10,13,16-pentaoxaoctadecanoate ion, a (methylthio)acetate ion, an (ethylthio)acetate ion, a 3-(methylthio)propanoate ion, a 3-[(2-methoxyethyl)thio]propanoate ion, or a 3-[(2-ethoxyethyl)thio]propanoate ion, and particularly preferably an acetate ion, a methoxyacetate ion, an acrylate ion, or a methacrylate ion, although the present invention is not limited by these ions.

The imidazolium carboxylate represented by Formula (1) is preferably an onium salt formed of a combination of one cation selected from the group consisting of 1,3-bis(2-methoxyethyl)imidazolium, 1,3-bis(3-methoxypropyl)imidazolium, 1,3-bis(3-ethoxypropyl)imidazolium, 1,3-bis(3-methylthiopropyl)imidazolium, 1,3-bis[3-(2-methoxyethoxy)propyl]imidazolium, 1-methoxypropyl-3-methylthiopropylimidazolium, 1,3-bis(2-methoxyethyl)-2-methylimidazolium, and 1,3-bis(3-methoxypropyl)-2-methylimidazolium with one anion selected from the group consisting of an acetate ion, a methoxyacetate ion, an acrylate ion, and a methacrylate ion.

More specifically, the onium salt represented by Formula (1) includes 1,3-bis(2-methoxyethyl)imidazolium acetate, 1,3-bis(3-methoxypropyl)imidazolium acetate, 1,3-bis(3-methoxypropyl)imidazolium acrylate, 1,3-bis(3-methoxypropyl)imidazolium methacrylate, 1,3-bis(3-ethoxypropyl)imidazolium acetate, 1-methoxypropyl-3-methylthiopropyl imidazolium acetate, 1,3-bis(3-methylthiopropyl)imidazolium acetate, 1,3-bis(2-methoxyethyl)-2-methyl imidazolium acetate, 1,3-bis(2-methoxyethyl)-2-methyl imidazolium acrylate, 1,3-bis(2-methoxyethyl)-2-methyl imidazolium methacrylate, 1,3-bis(3-methoxypropyl)-2-methyl imidazolium acetate, 1,3-bis(3-methoxypropyl)-2-methyl imidazolium acrylate, 1,3-bis(3-methoxypropyl)-2-methyl imidazolium methacrylate, 1,3-bis[3-(2-methoxyethoxy)propyl]imidazolium acetate, 1,3-bis[3-(2-methoxyethoxy)propyl]imidazolium acrylate, and 1,3-bis[3-(2-methoxyethoxy)propyl]imidazolium methacrylate. The onium salt is preferably 1,3-bis(3-methoxypropyl)imidazolium acetate, 1,3-bis(3-methoxypropyl)imidazolium acrylate, or 1,3-bis(3-methoxypropyl)imidazolium methacrylate, although the present invention is not limited by these compounds.

The present invention relates to a method for producing an onium salt represented by Formula (1), comprising the following steps (1), (2), and (3):
step (1) of obtaining a mixture containing the onium salt represented by Formula (1) and a carboxylic acid represented by the following Formula (3), the mixture being obtained according to a reaction scheme represented by the following Formula (2): 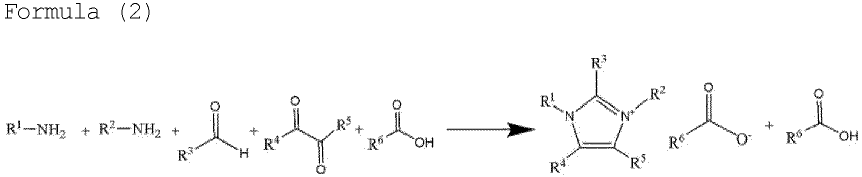 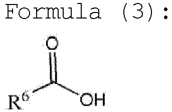 wherein R⁶ represents a hydrocarbon group optionally containing at least one heteroatom and may contain a double bond and/or a cyclic structure and/or a branched structure,
step (2) of adding an amine to the mixture containing the onium salt represented by Formula (1) and the carboxylic acid represented by Formula (3) obtained in step (1) to generate an amine-carboxylic acid salt formed of the amine and the carboxylic acid represented by Formula (3), and
step (3) of separating the onium salt represented by Formula (1) from the amine-carboxylic acid salt generated in step (2) to obtain the onium salt represented by Formula (1).

Step (1) is the step of obtaining a mixture containing the onium salt represented by Formula (1) and the carboxylic acid represented by Formula (3) according to a reaction scheme represented by the following Formula (2).

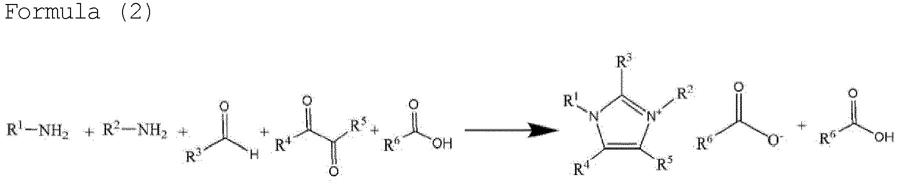

R¹, R², R³, R⁴, R⁵, and R⁶ in Formula (2) are respectively the same as R¹, R², R³, R⁴, R⁵, and R⁶ in Formula (1) .

The reaction of Formula (2) is explained. An amine compound represented by R²-NH₂ and/or R²-NH₂, an aldehyde compound represented by R³-CHO, a dicarbonyl compound represented by R⁴-C(=O)-C(=O)-R⁵, and a carboxylic acid represented by R⁶-COOH (Formula (3)) are reacted.

Specific examples of the amine compound represented by R¹-NH₂ or R²-NH₂ include 2-methoxyethylamine, 2-ethoxyethylamine, 2-propoxyethylamine, 2-isopropoxyethylamine, 2-allyloxyethylamine, 2-butoxyethylamine, 2-(1-methylpropoxy)ethylamine, 2-(2-methylpropoxy)ethylamine, 2-(1,1-dimethylethoxy)ethylamine, 2-pentyloxyethylamine, 2-neopentyloxyethylamine, 2-hexyloxyethylamine, 2-phenoxyethylamine, 2-heptyloxyethylamine, 2-octyloxyethylamine, 2-nonyloxyethylamine, 2-decyloxyethylamine, 2-cyclopentyloxyethylamine, 2-cyclohexyloxyethylamine, 2-(2-ethylhexan-1-yl)oxyethylamine, 2-(2,4,6-trimethylphenoxy)ethylamine, 2-(benzyloxy)ethylamine, 2-(p-methoxybenzyloxy)ethylamine, 2-(2-methoxyethoxymethoxy)ethylamine, 2-(2-benzyloxyethoxy)ethylamine, 2-(dimethylamino)ethylamine, 3-methoxypropylamine, 3-ethoxypropylamine, 3-propoxypropylamine, 3-allyloxypropylamine, 3-isopropoxypropylamine, 3-(1-methylpropoxy)propylamine, 3-(2-methylpropoxy)propylamine, 3-(1,1-dimethylethoxy)propylamine, 3-butoxypropylamine, 3-pentyloxypropylamine, 3-neopentyloxypropylamine, 3-hexyloxypropylamine, 3-cyclohexyloxypropylamine, 3-heptyloxypropylamine, 3-octyloxypropylamine, 3-nonyloxypropylamine, 3-(2-ethylhexan-1-yl)oxypropylamine, 3-(2,4,6-trimethylphenoxy)propylamine, 3-(2-methoxyethoxy)propylamine, 3-(dimethylamino)propylamine, 4-methoxybutylamine, 4-ethoxybutylamine, 4-propoxybutylamine, 4-isopropoxybutylamine, 4-allyloxybutylamine, 4-butoxybutylamine, 4-(1-methylpropoxy)butylamine, 4-(2-methylpropoxy)butylamine, 4-(1,1-dimethylethoxy)butylamine, 4-pentyloxybutylamine, 4-neopentyloxybutylamine, 4-hexyloxybutylamine, 4-cyclohexyloxybutylamine, 4-heptyloxybutylamine, 4-octyloxybutylamine, 4-(2-ethylhexan-1-yl)oxybutylamine, 5-methoxypentylamine, 5-ethoxypentylamine, 5-propoxypentylamine, 5-isopropoxypentylamine, 5-allyloxypentylamine, 5-butoxypentylamine, 5-(1-methylpropoxy)pentylamine, 5-(2-methylpropoxy)pentylamine, 5-(1,1-dimethylethoxy)pentylamine, 5-pentyloxypentylamine, 5-neopentyloxypentylamine, 5-hexyloxypentylamine, 5-cyclohexyloxypentylamine, 5-heptyloxypentylamine, 6-methoxyhexylamine, 6-ethoxyhexylamine, 6-propoxyhexylamine, 6-isopropoxyhexylamine, 6-allyloxyhexylamine, 6-butoxyhexylamine, 6-(1-methylpropoxy)hexylamine, 6-(2-methylpropoxy)hexylamine, 6-(1,1-dimethylethoxy)hexylamine, 6-pentyloxyhexylamine, 6-neopentyloxyhexylamine, 6-hexyloxyhexylamine, 6-cyclohexyloxyhexylamine, 2-methoxycyclohexyl-1-yl methylamine, (2-ethoxycyclohexan-1-yl)methylamine, (2-propoxycyclohexan-1-yl)methylamine, (2-isopropoxycyclohexan-1-yl)methylamine, (2-butoxycyclohexyl-1-yl)methylamine, [2-(1-methylpropoxy)cyclohexan-1-yl]methylamine, [2-(2-methylpropoxy)cyclohexan-1-yl]methylamine, [2-(1,1-dimethylethoxy)cyclohexan-1-yl]methylamine, (2-pentyloxycyclohexan-1-yl)methylamine, (2-neopentyloxycyclohexan-1-yl)methylamine, 2-methylthioethylamine, 3-methylthiopropylamine, 1-(methoxymethyl)propylamine, (oxolan-2-yl)methylamine, (furan-2-yl)methylamine, 2-[(furan-2-yl)methylthio]ethylamine, 2-(methylsulfonyl)ethylamine, (thiophen-2-yl)methylamine, p-methoxyphenylamine, p-ethoxyphenylamine, 3,4-methylenedioxyaniline, 3,4-methylenedioxybenzyl amine, and (7-oxabicyclo[2,2,1]heptan-2-yl)methylamine. The amine is preferably 2-methoxyethylamine, 3-methoxypropylamine, 3-ethoxypropylamine, 3-isopropoxypropylamine, 3-butoxypropylamine, 3-(2-methoxyethoxy)propylamine, 3-methylthiopropylamine, and 3-(dimethylamino)propylamine, and more preferably 2-methoxyethylamine, 3-methoxypropylamine, and 3-(2-methoxyethoxy)propylamine, although the present invention is not limited by these amines. The amine compound represented by R¹-NH₂ and the amine compound represented by R²-NH₂ may be identical or different.

Specific examples of the aldehyde compound represented by R³-CHO include formaldehyde, acetaldehyde, propionaldehyde, and butanal. Although the aldehyde compound is preferably formaldehyde or acetaldehyde, the present invention is not limited by these aldehyde compounds. Formaldehyde for use may be a cyclic oligomer or polymer capable of generating formaldehyde, such as paraformaldehyde, trioxane, or tetraoxane, in a reaction system.

The aldehyde compound represented by R³-CHO may be used in the form of an aqueous solution of the aldehyde compound or a solution of the aldehyde compound in an alcohol, such as methanol, butanol or 2-ethylhexanol.

Specific examples of the dicarbonyl compound represented by R⁴-C(=O)-C(=O)-R⁵ include glyoxal, methylglyoxal, diacetyl, 3,4-hexanedione, 2,3-pentanedione, 2,3-heptanedione, 5-methyl-2,3-hexanedione, 3-methyl-2,3-cyclopentanedione, 1,2-cyclohexanedione, 1-phenyl-1,2-propanedione, and dibenzoyl. The dicarbonyl compound is more preferably glyoxal, methylglyoxal, or diacetyl, and more preferably glyoxal.

The dicarbonyl compound represented by R⁴-C(=O)-C(=O)-R⁵ may be used in the form of an aqueous solution of the dicarbonyl compound or a solution of the dicarbonyl compound in an alcohol, such as methanol, butanol, or 2-ethylhexanol.

Specific examples of the carboxylic acid represented by R⁶-COOH (Formula (3)) include acetic acid, propionic acid, acrylic acid, propargylic acid, butyric acid, isobutyric acid, methacrylic acid, crotonic acid, tetrolic acid, cyclopropanecarboxylic acid, valeric acid, isovaleric acid, pivalic acid, angelic acid, tiglic acid, cyclobutanecarboxylic acid, caproic acid, cyclopentanecarboxylic acid, enanthic acid, sorbic acid, cyclohexanecarboxylic acid, benzoic acid, caprylic acid, 2-ethylhexylcarboxylic acid, toluic acid, 2-norbornane carboxylic acid, pelargonic acid, cinnamic acid, capric acid, adamantane carboxylic acid, geranic acid, undecylic acid, lauric acid, and naphthalene carboxylic acid. The carboxylic acid is preferably acetic acid, propionic acid, acrylic acid, propargylic acid, butyric acid, isobutyric acid, methacrylic acid, crotonic acid, tetrolic acid, valeric acid, isovaleric acid, pivalic acid, methoxyacetic acid, ethoxyacetic acid, propoxyacetic acid, (2-methoxyethoxy)acetic acid, (2-ethoxyethoxy)acetic acid, (2-propoxyethoxy)acetic acid, 3-(2-methoxyethoxy)propanoic acid, 3-(2-ethoxyethoxy)propanoic acid, 3-(2-propoxyethoxy)propanoic acid, 3-(3-methoxypropoxy)propanoic acid, 3-(3-ethoxypropoxy)propanoic acid, 3-(3-propoxypropoxy)propanoic acid, 3-[2-(2-methoxyethoxy)ethoxy]propanoic acid, 3-[2-(2-ethoxyethoxy)ethoxy]propanoic acid, 4,7,10,13-tetraoxatetradecanoic acid, 4,7,10,13-tetraoxapentadecanoic acid, 4,7,10,13,16-pentaoxaheptadecanoic acid, 4,7,10,13,16-pentaoxaoctadecanoic acid, (methylthio)acetic acid, (ethylthio)acetic acid, 3-(methylthio)propanoic acid, 3-[(2-methoxyethyl)thio]propanoic acid, or 3-[(2-ethoxyethyl)thio]propanoic acid, and particularly preferably acetic acid, methoxyacetic acid, acrylic acid, or methacrylic acid, although the present invention is not limited by these carboxylic acids.

The amount of the amine compounds represented by R¹-NH₂ and R²-NH₂ for use is preferably 0.1 to 10 mol and more preferably 0.5 to 3 mol in total of R¹-NH₂ and R²-NH₂, per mole of the dicarbonyl compound. When R¹ and R² are different, the ratio (molar ratio) of the two amine compounds (R¹-NH₂ and R²-NH₂) is not particularly limited and is within the range of R¹-NH₂:R²-NH₂=0:100 to 100:0. When R¹-NH₂:R²-NH₂=0:100 or 100:0, R¹=R². When R¹-NH₂:R²-NH₂=0:100 or 100:0 is not the case, the onium salt represented by Formula (1) obtained according to the reaction of Formula (2) is a mixture of the compounds represented by the following formulas. The onium salt represented by Formula (1) encompasses the compounds represented by the following Formula (1') and Formula (1''), and the mixture of these compounds may be used as the onium salt mixture described below. The mixture may be optionally purified to obtain a single desired compound, which may then be used as the onium salt represented by Formula (1).

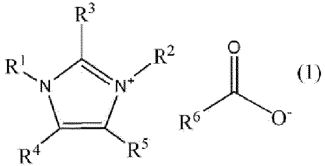

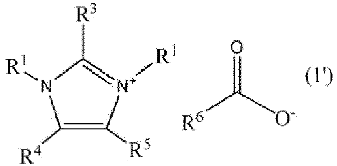

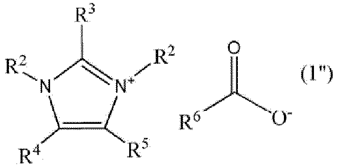

The aldehyde compound represented by R³-CHO may be used in the form of an aqueous solution of the aldehyde compound or a solution of the aldehyde compound in an alcohol, such as methanol, butanol, or 2-ethylhexanol. The amount of the aldehyde compound for use is preferably 0.1 to 10 mol, and more preferably 0.5 to 5 mol, per mole of the dicarbonyl compound.

The amount of the carboxylic acid represented by R⁶-COOH (Formula (3)) for use is preferably an excess amount per mole of the imidazole cation to be purified according to Formula (2). The amount of the carboxylic acid is preferably 1.1 to 10 mol, more preferably 1.2 to 5 mol, and particularly preferably 1.5 to 3 mol, per mole of the dicarbonyl compound. Accordingly, the reaction of Formula (2) produces a mixture containing the onium salt of Formula (1) and the carboxylic acid of Formula (3).

In the reaction of Formula (2), a solvent may or may not be used. A solvent for use can be any solvent that does not affect the reaction. Specific examples of solvents include aromatic hydrocarbon solvents, such as toluene, benzene, and xylene; hydrocarbon solvents, such as methylcyclohexane, cyclohexane, hexane, heptane, and octane; halogenated hydrocarbon solvents, such as dichloromethane and chloroform; ether solvents, such as diethyl ether, tetrahydrofuran, and 1,4-dioxane; alcohol solvents, such as methanol, ethanol, and 2-propanol; N,N-dimethylformamide, acetonitrile, and water, with alcohol solvents and water being preferred.

An onium salt represented by Formula (1) produced beforehand may also be used as a reaction solvent for the reaction of Formula (2). The solvent for use may be a mixture of any two or more solvents. The amount of the solvent for use is preferably 50 parts by mass or less, and more preferably 0.1 to 10 parts by mass, per part by mass of the dicarbonyl compound.

The reaction of Formula (2) may be optionally performed in an inert gas atmosphere that does not affect the reaction, such as nitrogen, argon, or helium.

For the reaction of Formula (2), the reaction pressure is typically atmospheric pressure. However, the reaction may be performed by placing the reaction system under an increased pressure of atmospheric pressure to 1 MPa, depending on the starting materials and/or solvent used.

For the reaction of Formula (2), the optimum reaction temperature varies depending on the starting materials, solvent, etc. used, but is preferably -10°C or higher, and more preferably 0°C to 100°C.

After completion of the reaction of Formula (2), the onium salt represented by Formula (1) may or may not be purified according to a known purification method. The purification method includes, but is not particularly limited to, concentration, liquid separation, recrystallization, dialysis, adsorption, and filtration.

Step (2) in the method for producing an onium salt represented by Formula (1) of the present invention is the step of adding an amine to the mixture containing the onium salt represented by Formula (1) and the carboxylic acid represented by Formula (3) obtained in step (1) to generate an amine-carboxylic acid salt formed of the amine and the carboxylic acid represented by Formula (3).

The amine added in step (2) is preferably an amine represented by the following Formula (4): 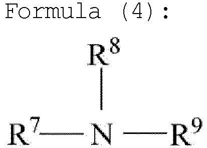 wherein
R⁷, R⁸, and R⁹ may be identical or different, and each represents a hydrogen atom or a C₁-C₁₈ hydrocarbon group,
the C₁-C₁₈ hydrocarbon group may contain a double bond and may contain a branched structure and/or a cyclic structure, and
the total number of carbon atoms contained in R⁷, R⁸, and R⁹ is 8 or greater.

In Formula (4), the number of carbon atoms of the hydrocarbon group represented by R⁷, R⁸, or R⁹ is 1 to 18, more preferably 1 to 12, and even more preferably 1 to 8.

The total number of carbon atoms contained in R⁷, R⁸, and R⁹ is 8 or greater, and preferably 12 or greater and 24 or less.

Specific examples of the hydrocarbon group represented by R⁷, R⁸, or R⁹ include a methyl group, an ethyl group, a propyl group, an isopropyl group, a butyl group, a sec-butyl group, an isobutyl group, a tert-butyl group, a pentyl group, a neopentyl group, a hexyl group, an octyl group, a 2-ethylhexyl group, a decyl group, a dodecyl group, and an octadecyl group. The isopropyl group, sec-butyl group, isobutyl group, tert-butyl group, neopentyl group, and 2-ethylhexyl group each have a branched structure.

Specific examples of the hydrocarbon group containing a double bond represented by R⁷, R⁸, or R⁹ include a vinyl group, an ethynyl group, a propenyl group, an isopropenyl group, an allyl group, a propargyl group, a butenyl group, a crotyl group, a butadienyl group, and an oleyl group.

Specific examples of the hydrocarbon group having a cyclic structure represented by R⁷, R⁸, or R⁹ include a cyclopropyl group, a cyclobutyl group, a cyclopentyl group, a cyclohexyl group, a norbornyl group, an adamantyl group, a phenyl group, a benzyl group, a tolyl group, a styryl group, and a 2,4,6-trimethylphenyl group. The cyclopropyl group, cyclobutyl group, cyclopentyl group, cyclohexyl group, norbornyl group, and adamantyl group each have a branched structure.

R⁷, R⁸, and R⁹ are preferably a hydrogen atom, a methyl group, an oleyl group, a 2-ethylhexyl group, a cyclohexyl group, an octadecyl group, or an octyl group.

Specific examples of the amine represented by Formula (4) include tripropylamine, tributylamine, tripentylamine, trihexylamine, triheptylamine, trioctylamine, trinonylamine, tridecylamine, oleylamine, di-2-ethylhexylamine, dicyclohexylamine, methyloctadecylamine, dimethyloctadecylamine, and trioctylamine.

The amount of the amine added to the mixture containing the onium salt represented by Formula (1) and the carboxylic acid represented by Formula (3) is 1 equivalent or more, preferably 1 to 1.5 equivalents, more preferably 1 to 1.2 equivalents, and particularly preferably 1 to 1.1 equivalents, per equivalent of the carboxylic acid. An amine-carboxylic acid salt composed of the amine added and the carboxylic acid of Formula (3) is generated, and a mixture of the onium salt of Formula (1), the amine-carboxylic acid salt, and the remaining amine is obtained.

The amine may be added in the form of an amine solution prepared by diluting the amine with a solvent. Examples of solvents include chloroform, dichloromethane, 2-ethylhexanol, cyclohexanol, 1-octanol, and ethyl acetate, among which chloroform, 2-ethylhexanol, cyclohexanol, and 1-octanol are preferred. The concentration of the amine solution is preferably 0.01 g/L to 10.0 g/L, and more preferably 0.1 g/L to 5.00 g/L.

The reaction conditions for generating an amine-carboxylic acid salt by adding an amine to a mixture containing an onium salt represented by Formula (1) and a carboxylic acid represented by Formula (3) are as follows: the temperature is 0°C to 100°C, preferably 25°C to 80°C; in regards to the reaction time, after the amine is added, the mixture is preferably stirred for 0.5 hours to 3 hours, and more preferably 1 hour to 2 hours.

In a preferred embodiment of the present invention, the mixture in step 1 further contains water and a solvent. The solvent may be a solvent for diluting the amine to be added, or may be a solvent added alone to the mixture in step 1 without being used to dilute the amine. The solvent contained in the mixture is preferably a solvent that undergoes phase separation from water. Examples of solvents that undergo phase separation from water include hexane, heptane, toluene, chloroform, dichloromethane, 2-ethylhexanol, cyclohexanol, 1-octanol, and ethyl acetate. The solvent is preferably chloroform, 2-ethylhexanol, cyclohexanol, or 1-octanol.

When the mixture in step 1 contains water and the solvent above, the added amine forms an amine-carboxylic acid salt and is extracted primarily into the solvent layer. On the other hand, the onium salt represented by Formula (1) is present in the aqueous layer. Thus, separating the solvent layer enables separation of the amine-carboxylic acid salt, thereby giving an aqueous layer containing the onium salt represented by Formula (1) in which the amount of the carboxylic acid represented by Formula (3) is reduced or the carboxylic acid is almost completely removed. It is difficult to remove the carboxylic acid of Formula (3) present together with the onium salt of Formula (1) by evaporation. However, water in the aqueous layer containing the onium salt of Formula (1) can be easily removed by evaporation to obtain the onium salt of Formula (1). The mixture containing the onium salt represented by Formula (1) and the carboxylic acid represented by Formula (3) before undergoing the production method or purification method of the present invention exhibits greatly reduced solubility of cellulose due to the presence of the carboxylic acid represented by Formula (3). However, performing the production method or purification method of the present invention significantly improves the solubility of cellulose. For example, the solubility of cellulose can be greatly improved by removing 50% or more, 60% or more, 65% or more, 70% or more, 75% or more, 80% or more, 85% or more, 90% or more, 95% or more, or 100% of the carboxylic acid represented by Formula (3) from a mixture containing the onium salt represented by Formula (1) and the carboxylic acid represented by Formula (3) .

Step (3) is the step of separating the onium salt represented by Formula (1) from the amine-carboxylic acid salt generated in step (2). Examples of separation methods include liquid separation and crystallization.

The separation in step (3) provides a separated liquid containing the onium salt represented by Formula (1). When the obtained separated liquid contains residual amine, the separated liquid may be heated and/or depressurized to distill off the residual amine.

When the amine is added in the form of an amine solution prepared by diluting the amine with a solvent, and the solvent is contained in the separated liquid containing the onium salt represented by Formula (1) separated in step (3), the separated liquid may be heated and/or depressurized to distill off the solvent.

In the present invention, the onium salt represented by Formula (1) may be one type or a mixture of at least two types. Specific examples of mixtures of at least two types of the onium salt include a mixture of 1,3-dimethylimidazolium acetate and 1,3-diethylimidazolium acetate, a mixture of 1,3-dimethylimidazolium acetate and 1-ethyl-3-methylimidazolium acetate, a mixture of 1,3-diethylimidazolium acetate and 1-ethyl-3-methylimidazolium acetate, and a mixture of 1,3-dimethylimidazolium acetate, 1,3-diethylimidazolium acetate, and 1-ethyl-3-methylimidazolium acetate.

In the present invention, the mixture containing the onium salt represented by Formula (1) and the carboxylic acid represented by Formula (3) may further contain water and/or a hydrophilic solvent. The hydrophilic solvent refers to a solvent that can be dissolved in or is miscible with water, and specific examples include C₁-C₄ lower alcohols, such as methanol, ethanol, propanol, and ethylene glycol.

The content of water and/or the hydrophilic solvent is 0.1 mass% to 90 mass% or 1 mass% to 70 mass%, and preferably 5 mass% to 50 mass%, based on the total amount of the mixture containing the onium salt represented by Formula (1), the carboxylic acid represented by Formula (3), and water and/or the hydrophilic solvent taken as 100 mass%.

One embodiment of the present invention relates to a method for purifying an onium salt represented by Formula (1) comprising removing a carboxylic acid represented by Formula (3) from a mixture containing the onium salt represented by Formula (1) and the carboxylic acid represented by Formula (3),
the method comprising the following steps (1), (2), and (3) :
step (1) of obtaining a mixture containing the onium salt represented by Formula (1) and the carboxylic acid represented by Formula (3), the mixture being obtained according to a reaction scheme represented by Formula (2),
step (2) of adding an amine to the mixture containing the onium salt represented by Formula (1) and the carboxylic acid represented by Formula (3) obtained in step (1) to generate an amine-carboxylic acid salt formed of the amine and the carboxylic acid represented by Formula (3), and
step (3) of separating the onium salt represented by Formula (1) from the amine-carboxylic acid salt generated in step (2) to obtain the onium salt represented by Formula (1).

In the method for purifying an onium salt of the present invention, the onium salt represented by Formula (1) is the same as the onium salt represented by Formula (1) in the method for producing an onium salt described above. In the onium salt represented by Formula (1) in the method for purifying an onium salt of the present invention, the imidazolium cation represented by Formula (1-1) is the same as the imidazolium cation represented by Formula (1-1) in the method for producing an onium salt described above. In the onium salt represented by Formula (1) in the method for purifying an onium salt of the present invention, the carboxylate anion represented by Formula (1-2) is the same as the carboxylate onion represented by Formula (1-2) in the method for producing an onium salt described above.

In the method for purifying an onium salt of the present invention, the carboxylic acid represented by Formula (3) is the same as the carboxylic acid represented by Formula (3) in the method for producing an onium salt described above.

In the method for purifying an onium salt of the present invention, the mixture containing the onium salt represented by Formula (1) and the carboxylic acid represented by Formula (3) obtained according to the reaction of Formula (2) in step (1) of the purification method is the same as the mixture containing the onium salt represented by Formula (1) and the carboxylic acid represented by Formula (3) obtained according to the reaction of Formula (2) in step (1) of the method for producing an onium salt described above.

In the method for purifying an onium salt of the present invention, the mixture containing the onium salt represented by Formula (1) and the carboxylic acid represented by Formula (3) obtained according to the reaction of Formula (2) in step (1) of the purification method is a mixture in which the carboxylic acid represented by Formula (3) used in the reaction of Formula (2) remains with the onium salt represented by Formula (1). The amount of the remaining carboxylic acid of Formula (3) is typically 1.5 equivalents or less per equivalent of the onium salt of Formula (1).

In the method for purifying an onium salt of the present invention, step (2) is the same as step (2) in the method for producing an onium salt described above, and is a step of adding an amine to a mixture containing the onium salt represented by Formula (1) and the carboxylic acid represented by Formula (3) to produce an amine-carboxylic acid salt formed of the amine and the carboxylic acid represented by Formula (3).

The amine added in step (2) of the method for purifying an onium salt of the present invention is preferably the amine represented by Formula (4) in the method for producing an onium salt described above.

In step (2) of the method for purifying an onium salt of the present invention, the amount, and the preferred amount, of the amine added to the mixture containing the onium salt represented by Formula (1) and the carboxylic acid represented by Formula (3) are the same as the amount, and the preferred amount, of the amine added in the method for producing an onium salt described above. Step (2) of the method for purifying an onium salt of the present invention generates an amine-carboxylic acid salt formed of the added amine and the carboxylic acid of Formula (3) as in step (2) of the method for producing an onium salt described above, providing a mixture of the onium salt represented by Formula (1), the amine-carboxylic acid salt, and the remaining amine.

The amine added in step (2) of the method for purifying an onium salt of the present invention may be an amine solution prepared by diluting the amine with a solvent, as with the amine added in step (2) of the method for producing an onium salt described above. The solvent may be the same as that used in step (2) of the production method described above, and the concentration of the amine solution may be the same as that used in step (2) of the production method.

In step (2) of the method for purifying an onium salt of the present invention, the reaction conditions for generating an amine-carboxylic acid salt by adding an amine to a mixture containing the onium salt represented by Formula (1) and the carboxylic acid represented by Formula (3), such as the temperature conditions and reaction time, are the same as the reaction conditions in step (2) of the production method above.

In the method for purifying an onium salt of the present invention, step (3) is the same as step (3) in the method for producing an onium salt above, and is a step of separating the onium salt represented by Formula (1) from the amine-carboxylic acid salt produced in step (2). The separation method is the same as the separation method used in step (3) of the method for producing an onium salt described above.

The separation in step (3) in the method for purifying an onium salt of the present invention provides a separated liquid containing the onium salt represented by Formula (1). When the obtained separated liquid contains residual amine and/or the solvent of the amine solution, the obtained separated liquid can be heated and/or depressurized to distill off the remaining amine and/or solvent in the same manner as in step (3) of the method for producing an onium salt described above.

In the method for purifying an onium salt of the present invention, the onium salt represented by Formula (1) may be one type or a mixture of at least two types. The mixture of at least two types is the same as the mixture of at least two types of the onium salt represented by Formula (1) in the method for producing an onium salt described above.

In the method for purifying an onium salt of the present invention, the mixture containing the onium salt represented by Formula (1) and the carboxylic acid represented by Formula (3) may further contain water and/or a hydrophilic solvent, as in the method for producing an onium salt described above. The hydrophilic solvent may be the same as the hydrophilic solvent used in the method for producing an onium salt described above, and the content of water and/or a hydrophilic solvent is also the same as the content of water and/or a hydrophilic solvent in the method for producing an onium salt described above.

The onium salt produced according to the method of the present invention can be used, for example, as a solvent for dissolving cellulose or as a carbon dioxide absorbent.

### Examples

The following describes the present invention in detail with reference to Examples. However, the present invention is not limited to these Examples in any way.

### Example 1

A 1 L four-necked reactor purged with nitrogen was charged with 113 g (1.40 mol) of a 37 wt% aqueous formaldehyde solution (manufactured by Junsei Chemical Co., Ltd.), 126 g (2.10 mol) of acetic acid (manufactured by Junsei Chemical Co., Ltd.), 250 g (2.81 mol) of 3-methoxypropylamine (manufactured by Tokyo Chemical Industry Co., Ltd.), and 202 g (1.40 mol) of a 40 wt% aqueous glyoxal solution (manufactured by Tokyo Chemical Industry Co., Ltd.). The obtained mixture was heated to 40°C and stirred for 3 hours to synthesize 691 g of an aqueous solution of 1,3-bis (3-methoxypropyl) imidazolium acetate ([(MeOPr)₂Im][AcO]). The synthesized substance is referred below to as the "reaction solution." The reaction solution was subjected to ¹H NMR analysis to determine the integral value of the peaks derived from acetate ions and acetic acid at 1.55-1.65 ppm. The integral value of the peaks at 1.55 to 1.65 ppm was 4.66 based on peaks of 1.94 to 2.14 ppm (quin., 4H).

To 10.0 g of the reaction solution, 5.57 g of ion-exchanged water, 10.0 g of chloroform (manufactured by Junsei Chemical Co., Ltd.), and the individual amine (0.02 mol) shown in Evaluation Examples 1 to 4 in Table 1 were added, and the mixture was stirred and subjected to liquid separation. About 3 drops of the aqueous layer were added to about 1 g of DMSO-d6 to prepare a sample for ¹H NMR analysis. The integral value of the peaks at 1.55 to 1.65 ppm was determined based on peaks of 1.94-2.14 ppm (quin., 4H), and the removal rate (%) of acetic acid was calculated using the following formula. Acetic acid removal rate (%) = (1 - (integral value - 3.00)/(4.66 - 3.00)) × 100

In Evaluation Example 2, because the peaks derived from dicyclohexylamine overlapped with the peaks derived from acetate ions and acetic acid, the removal rate was calculated after removing the 2H component derived from the amine.

Table 1 shows the results. Because the onium salts obtained using the amines of Evaluation Examples 1 to 3 were those from which acetic acid was removed, all of them are capable of improving the solubility of cellulose compared with the onium salts before the removal of acetic acid. However, because of its low acetic acid removal rate, the onium salt obtained by using the amine of Evaluation Example 4 was presumed to hardly improve the solubility of cellulose.

**Table 1**

| | Amine | Removal Rate |
|---|---|---|
| Evaluation Example 1 | Bis (2-ethylhexyl)amine | 84.9 |
| Evaluation Example 2 | Dicyclohexylamine | 75.3 |
| Evaluation Example 3 | Dibutylamine | 54.2 |
| Evaluation Example 4 | Triethylamine | 5.4 |

### Example 2

To 10.0 g of the reaction solution obtained in Example 1, 5.57 g of ion-exchanged water, 10.0 g of 2-ethylhexanol (manufactured by Junsei Chemical Co., Ltd.), and the individual amine (0.02 mol) of Evaluation Examples 5 and 6 shown in Table 2 were added, and the mixture was stirred and subjected to liquid separation. About 3 drops of the aqueous layer were added to about 1 g of DMSO-d6 to prepare a sample for ¹H NMR analysis. The integral value of the peaks derived from acetate ions and acetic acid at 1.55-1.65 ppm was determined based on peaks of 1.94-2.14 ppm (quin., 4H), and the removal rate (%) of acetic acid was calculated using the following formula. As described in Example 1, the integral value of the peaks at 1.55-1.65 ppm of the reaction solution was 4.66. Acetic acid removal rate (%) = (1 - (integral value - 3.00)/(4.66 - 3.00)) × 100

In Evaluation Example 6, because the peaks derived from dicyclohexylamine overlapped with the peaks derived from acetate ions and acetic acid, the removal rate was calculated after removing the 2H component derived from the amine.

Table 2 shows the results. Because the onium salts obtained using the amines of Evaluation Examples 5 to 7 were those from which acetic acid was removed, all of them are capable of improving the solubility of cellulose compared with the onium salts before the removal of acetic acid.

**Table 2**

| | Amine | Removal Rate |
|---|---|---|
| Evaluation Example 5 | Bis(2-ethylhexyl)amine | 84.9 |
| Evaluation Example 6 | Dicyclohexylamine | 77.1 |
| Evaluation Example 7 | Dibutylamine | 60.8 |

### Example 3

A 30 mL three-necked reactor purged with nitrogen was charged with 1.69 g (0.21 mol) of a 37 wt% aqueous formaldehyde solution (manufactured by Junsei Chemical Co., Ltd.), 1.83 g (0.30 mol) of acetic acid (manufactured by Junsei Chemical Co., Ltd.), 1.63 g (0.21 mol) of a 40% solution of methylamine in methanol (manufactured by Tokyo Chemical Industry Co., Ltd.), 1.32 g (0.20 mol) of a 70% aqueous ethylamine solution (manufactured by Tokyo Chemical Industry Co., Ltd.), and 2.99 g (0.21 mol) of a 40 wt% aqueous glyoxal solution (manufactured by Tokyo Chemical Industry Co., Ltd.). The obtained mixture was heated to 40°C and stirred for 3 hours to synthesize 8.97 g of an aqueous solution of a mixture of 1,3-dimethylimidazolium acetate, 1,3-diethylimidazolium acetate, and 1-ethyl-3-methylimidazolium acetate.

The reaction solution was subjected to ¹H NMR analysis to determine the integral value of the peaks derived from acetate ions and acetic acid at 1.55-1.65 ppm. The integral of the peaks at 1.55 to 1.67 ppm was 4.70 based on peaks of 7.55 to 7.75 ppm (m, 2H).

To the reaction solution, 5.22 g of ion-exchanged water, 4.77 g of 2-ethylhexanol, and 2.72 g (0.21 mol) of di-2-ethylhexylamine were added, and the mixture was stirred and subjected to liquid separation. About 3 drops of the aqueous layer were added to about 1 g of DMSO-d6 to prepare a sample for ¹H NMR analysis. The integral value of the peaks at 1.55 to 1.67 ppm was determined based on peaks of 7.55 to 7.75 ppm (m, 2H), and the removal rate (%) of acetic acid was calculated using the following formula. Acetic acid removal rate (%) = (1 - (integral value - 3.00)/(4.70 - 3.00)) × 100

Table 3 shows the results. Because the onium salt obtained using the amine of Evaluation Example 8 was one from which acetic acid was removed, the onium salt is capable of improving the solubility of cellulose compared with the onium salt before the removal of acetic acid.

**Table 3**

| | Amine | Removal Rate |
|---|---|---|
| Evaluation Example 8 | Di-2-ethylhexylamine | 96.5 |

### Example 4

A 300 L four-necked reactor purged with nitrogen was charged with 28.40 g (0.35 mol) of a 37 wt% aqueous formaldehyde solution (manufactured by Junsei Chemical Co., Ltd.), 31.59 g (0.53 mol) of acetic acid (manufactured by Junsei Chemical Co., Ltd.), 62.31 g (0.70 mol) of 3-methoxypropylamine (manufactured by Tokyo Chemical Industry Co., Ltd.), and 50.45 g (0.35 mol) of a 40 wt% aqueous glyoxal solution (manufactured by Tokyo Chemical Industry Co., Ltd.). The obtained mixture was heated to 40°C and stirred for 3 hours to synthesize 172 g of an aqueous solution of 1,3-bis(3-methoxypropyl)imidazolium acetate ([(MeOPr)₂Im][AcO]). The synthesized substance is referred below to as the "reaction solution." The reaction solution was concentrated and subjected as is to ¹H NMR analysis and a cellulose dissolution test. The results of ¹H NMR analysis indicate that the integral value of the peaks derived from acetate ions and acetic acid based on peaks of 1.94 to 2.14 ppm (quin., 4H) was 4.49.

### Removal of Acetic Acid

Ion-exchanged water (8.36 g), chloroform (15.0 g) (manufactured by Junsei Chemical Co., Ltd.), and individual amine (0.03 mol) shown in Table 4 were added to the reaction solution (15.0 g), and the mixture was stirred and allowed to stand (80°C, 1 hour each). After liquid separation, the lower layer was concentrated and subjected to ¹H NMR analysis and a cellulose dissolution test. These are referred below to as "Evaluation Examples 9 to 11" and "Comparative Example 2." In Comparative Example 1, the cellulose dissolution test was performed in the same manner as above, except that no amine was used.

In the NMR analysis, the integral value of the peaks derived from acetic acid was confirmed. The integral value of the peaks at 1.55 to 1.65 ppm was determined based on peaks of 1.94 to 2.14 ppm (quin., 4H), and the removal rate (%) of acetic acid was calculated from the integral value. The formula is as follows: Acetic acid removal rate (%) = (1 - (integral value - 3.00)/(4.49 - 3.00)) × 100

In Evaluation Example 10, because the peaks derived from dicyclohexylamine overlapped with the peaks derived from acetic acid, the removal rate was calculated after removing the 2H component derived from the amine. Table 5 shows the results.

### Dissolution Test

A dissolution test was performed to confirm whether microcrystalline cellulose (10 wt% vs. concentrated solution) (Avicel (registered trademark) PH-101, manufactured by Sigma-Aldrich Co. LLC) could be dissolved at 25°C.

The concentrated solution of the reaction solution (Comparative Example 1) and the concentrated solutions (1.0 g) of Evaluation Examples 9 to 11 and Comparative Example 2 were each placed in a test tube, and microcrystalline cellulose (10 wt% vs. concentrated solution) was added at 25°C and stirred. After 24 hours, whether the microcrystalline cellulose was dissolved was visually examined. The samples in which the crystalline cellulose was completely dissolved were marked with "√" and the samples in which the crystalline cellulose was not completely dissolved were marked with "X." Table 5 shows the results of dissolution.

### Example 5

### Acetic Acid Removal

Ion-exchanged water (8.36 g), 2-ethylhexanol (15.0 g) (manufactured by Junsei Chemical Co., Ltd.), and the individual amine (0.03 mol) shown in Table 1 were added to the reaction liquid (15.0 g) obtained in Example 4, and the mixture was stirred and allowed to stand (80°C, 1 hour each). After liquid separation, the lower layer was concentrated and subjected to ¹H NMR analysis and a dissolution test. These are referred below to as "Evaluation Examples 12 to 14."

In the NMR analysis, the integral value of the peaks derived from acetic acid was confirmed. The integral value of the peaks at 1.55 to 1.65 ppm was determined based on 1.94 to 2.14 ppm (quin., 4H), and the removal rate (%) of acetic acid was calculated from the integral value. The formula is as follows: Acetic acid removal rate (%) = (1 - (integral value - 3.00)/(4.49 - 3.00)) × 100

In Evaluation Example 13, because the peaks derived from dicyclohexylamine overlapped with the peaks derived from acetic acid, the removal rate was calculated after removing the 2H component derived from the amine. Table 6 shows the results.

### Dissolution Test

A dissolution test was performed to confirm whether microcrystalline cellulose (10 wt% vs. concentrated solution) (Avicel (registered trademark) PH-101, manufactured by Sigma-Aldrich Co. LLC) could be dissolved at 25°C.

The concentrated solutions (1.0 g) of Evaluation Examples 12 to 14 were each placed in a test tube, and microcrystalline cellulose (10 wt% vs. concentrated solution) was added at 25°C and stirred. After 24 hours, whether the microcrystalline cellulose was dissolved was visually examined. The samples in which the crystalline cellulose was completely dissolved were marked with "√" and the samples in which the crystalline cellulose was not completely dissolved were marked with "X." Table 6 shows the results of dissolution.

**Table 4**

| Amine | Source |
|---|---|
| Bis(2-ethylhexyl)amine | Koei Chemical Co., Ltd. |
| Dicyclohexylamine | Tokyo Chemical Industry Co., Ltd. |
| Dibutylamine | Tokyo Chemical Industry Co., Ltd. |
| Triethylamine | Tokyo Chemical Industry Co., Ltd. |

**Table 5**

| | Amine | Removal Rate (%) | Dissolution of 10 wt% Cellulose |
|---|---|---|---|
| Comparative Example 1 | - | 0 | X |
| Comparative Example 2 | Triethylamine | 2.7 | X |
| Evaluation Example 9 | Bis(2-ethylhexyl)amine | 91.3 | √ |
| Evaluation Example 10 | Dicyclohexylamine | 79.9 | √ |
| Evaluation Example 11 | Dibutylamine | 46.3 | √ |

**Table 6**

| | Amine | Removal Rate (%) | Dissolution of 10 wt% Cellulose |
|---|---|---|---|
| Evaluation Example 12 | Bis(2-ethylhexyl)amine | 100.0 | √ |
| Evaluation Example 13 | Dicyclohexylamine | 78.5 | √ |
| Evaluation Example 14 | Dibutylamine | 57.7 | √ |

### Industrial Applicability

The present invention provides a method for producing an onium salt that is free of carboxylic acid or an onium salt with a decreased carboxylic acid content to such an extent that sufficient solubility of cellulose in the onium salt is achieved. The present invention also provides a method for purifying an onium salt, by removing carboxylic acid contained in the onium salt or decreasing the content of carboxylic acid to such an extent that sufficient solubility of cellulose in the onium salt is achieved.

## Claims

1. A method for producing an onium salt represented by the following Formula (1): 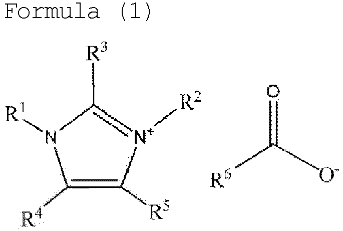 wherein
R¹, R², R³, R⁴, and R⁵ may be identical or different, and each represents a hydrogen atom or a hydrocarbon group optionally containing at least one heteroatom,
R¹, R², R³, R⁴, and R⁵ may contain a branched and/or cyclic structure and/or a double bond,
some or all of R¹, R², R³, R⁴, and R⁵, taken together with the nitrogen and/or carbon atoms to which R¹, R², R³, R⁴, and R⁵ are bonded, may form a cyclic structure, and
R⁶ represents a hydrocarbon group optionally containing at least one heteroatom and may contain a double bond and/or a cyclic structure and/or a branched structure,
the method comprising the following steps (1), (2) and (3) :
step (1) of obtaining a mixture containing the onium salt represented by Formula (1) and a carboxylic acid represented by the following Formula (3), the mixture being obtained according to a reaction scheme represented by the following Formula (2): 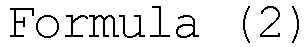 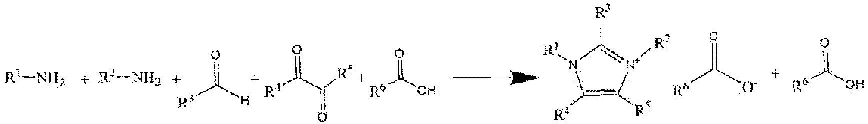 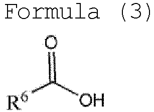 wherein R⁶ represents a hydrocarbon group optionally containing at least one heteroatom and may contain a double bond and/or a cyclic structure and/or a branched structure,
step (2) of adding an amine to the mixture containing the onium salt represented by Formula (1) and the carboxylic acid represented by Formula (3) obtained in step (1) to generate an amine-carboxylic acid salt formed of the amine and the carboxylic acid represented by Formula (3), and
step (3) of separating the onium salt represented by Formula (1) from the amine-carboxylic acid salt generated in step (2) to obtain the onium salt represented by Formula (1).

2. The method for producing an onium salt according to claim 1, wherein the amine added in step (2) is an amine represented by the following Formula (4): 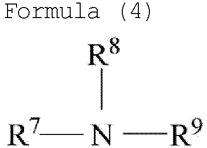 wherein
R⁷, R⁸, and R⁹ may be identical or different, and each represents a hydrogen atom or a C₁-C₁₈ hydrocarbon group,
the C₁-C₁₈ hydrocarbon group may contain a double bond and may contain a branched structure and/or a cyclic structure, and
the total number of carbon atoms contained in each of R⁷, R⁸, and R⁹ is 8 or greater.

3. The method for producing an onium salt according to 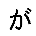 claim 1, wherein the onium salt represented by Formula (1) is of at least two types.

4. The method for producing an onium salt according to claim 1, wherein the mixture containing the onium salt represented by Formula (1) and the carboxylic acid represented by Formula (3) further contains water and/or a hydrophilic solvent.

5. The method for producing an onium salt according to claim 1, wherein the amine added in step (2) is an amine solution prepared by diluting the amine with a solvent.

6. A method for purifying an onium salt represented by the following Formula (1), comprising removing a carboxylic acid represented by the following Formula (3) from a mixture containing the onium salt represented by Formula (1) and the carboxylic acid represented by Formula (3), 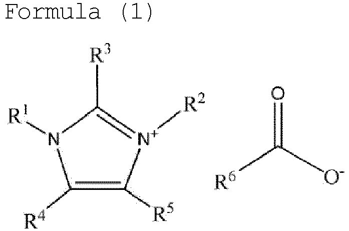 wherein
R¹, R², R³, R⁴, and R⁵ may be identical or different, and each represents a hydrogen atom or a hydrocarbon group optionally containing at least one heteroatom,
R¹, R², R³, R⁴, and R⁵ may contain a branched and/or cyclic structure and/or a double bond,
some or all of R¹, R², R³, R⁴, and R⁵, taken together with the nitrogen and/or carbon atoms to which R¹, R², R³, R⁴, and R⁵ are bonded, may form a cyclic structure, and
R⁶ represents a hydrocarbon group optionally containing at least one heteroatom and may contain a double bond and/or a cyclic structure and/or a branched structure,
the method comprising the following steps (1), (2) and (3) :
step (1) of obtaining a mixture containing the onium salt represented by Formula (1) and the carboxylic acid represented by Formula (3), the mixture being obtained according to a reaction scheme represented by the following Formula (2): 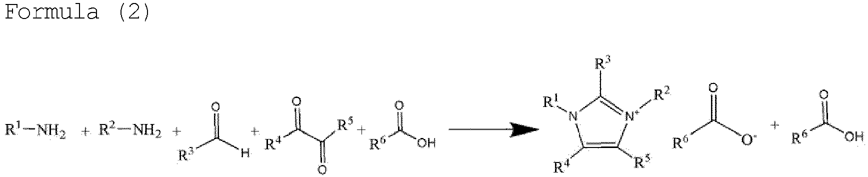 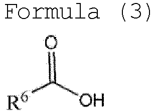 wherein R⁶ represents a hydrocarbon group optionally containing at least one heteroatom and may contain a double bond and/or a cyclic structure and/or a branched structure,
step (2) of adding an amine to the mixture containing the onium salt represented by Formula (1) and the carboxylic acid represented by Formula (3) obtained in step (1) to generate an amine-carboxylic acid salt formed of the amine and the carboxylic acid represented by Formula (3), and
step (3) of separating the onium salt represented by Formula (1) from the amine-carboxylic acid salt generated in step (2) to obtain the onium salt represented by Formula (1).

7. The method for purifying an onium salt according to claim 6, wherein the amine added in step (2) is an amine represented by the following Formula (4): 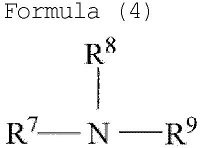 wherein
R⁷, R⁸, and R⁹ may be identical or different, and each represents a hydrogen atom or a C₁-C₁₈ hydrocarbon group,
the C₁-C₁₈ hydrocarbon group may contain a double bond and may contain a branched structure and/or a cyclic structure, and
the total number of carbon atoms contained in each of R⁷, R⁸, and R⁹ is 8 or greater.

8. The method for purifying an onium salt according to claim 6, wherein the onium salt represented by Formula (1) is of at least two types.

9. The method for purifying an onium salt according to claim 6, wherein the mixture containing the onium salt represented by Formula (1) and the carboxylic acid represented by Formula (3) further contains water and/or a hydrophilic solvent.

10. The method for purifying an onium salt according to claim 6, wherein the amine added in step (2) is an amine solution prepared by diluting the amine with a solvent.
